(19) Europäisches Patentamt
European Patent Office
Office européen des brevets

(11) Numéro de publication: **0 069 012**
**B1**

(12) **FASCICULE DE BREVET EUROPEEN**

(45) Date de publication du fascicule du brevet: **10.10.84**

(51) Int. Cl.[3]: **C 07 D 211/14**, C 07 D 295/12, C 07 D 211/46, A 61 K 31/445

(21) Numéro de dépôt: **82401154.8**

(22) Date de dépôt: **23.06.82**

(54) **Dérivés de benzoyl-phényl-pipéridine, procédé de préparation et application en thérapeutique.**

(30) Priorité: **29.06.81 FR 8112745**

(43) Date de publication de la demande: **05.01.83 Bulletin 83/1**

(45) Mention de la délivrance du brevet: **10.10.84 Bulletin 84/41**

(84) Etats contractants désignés: **AT BE CH DE FR GB IT LI LU NL SE**

(56) Documents cités:
**FR - A - 1 375 300**
**FR - A - 1 526 708**
**FR - A - 1 530 393**
**FR - A - 2 070 102**
**FR - A - 2 238 480**
**GB - A - 2 056 447**
**US - A - 3 668 199**
**US - A - 4 021 563**

**Arch.Pharm. 312, pages 219-230 (1979)**

(73) Titulaire: **SOCIETE DE RECHERCHES INDUSTRIELLES S.O.R.I. Société anonyme dite:, 3 rue de Citeaux, F-75012 Paris (FR)**

(72) Inventeur: **Majoie, Bernard, 71 rue Montchapet, F-21000 Dijon (FR)**
Inventeur: **Bellamy, François, Rue Basse Cidex 11, F-21910 Saulon-la-Rue (FR)**
Inventeur: **Dodey, Pierre, 21 rue des Buttes, F-21000 Dijon (FR)**
Inventeur: **Robin, Jacques, 19 rue de la Corvée, F-21000 Dijon (FR)**

(74) Mandataire: **Richebourg, Michel François et al, Cabinet Beau de Loménie 55, rue d'Amsterdam, F-75008 Paris (FR)**

EP 0 069 012 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

La présente invention concerne en tant que produits industriels nouveaux des dérivés appartenant à la famille des benzoyl-phényl-pipéridines, à savoir des dérivés de 2-pipéridinobenzophénone de formule I ci-après. Elle concerne également leur procédé de préparation et leur aplication en thérapeutique, notamment en tant qu'agents immunostimulants et immunoadjuvants.

On sait qu'un certain nombre de dérivés de benzoyl-phényl-pipéridine (qui ne répondent pas à la formule I ci-après) ont déjà été décrits. On connaît en particulier la [2-(1-pipéridinyl)-phényl]-(2-amino-5-chlorophényl)-méthanone de FR-A-1 375 300 et FR-A-1 403 939 en tant que produit intermédiaire de synthèse dans la préparation de benzodiazépines, les [2-amino-5-(1-pipéridinyl)-phényl]-(phényl)-méthanone et [2-nitro-5-(1-pipéridinyl)-phényl]-(phényl)-méthanone de FR-A-1 350 325 en tant que produits intermédiaires de synthèse dans la préparation de benzodiazépines, les [3-amino-4-(1-pipéridinyl)-phényl]-(phényl)-méthanone et [3-nitro-4-(1-pipéridinyl)-phényl]-(phényl)-méthanone de FR-A-2 238 480 et FR-A-2 238 483) où ils ont été préconisés en tant qu'agents inducteurs de L'enzyme microsomale hépatique et agents antipyrétiques, et la [3,5-dinitro-2-(1-pipéridinyl)-phényl]-(phényl)-méthanone de l'article de LOUDON et al., J. Chem. Soc., 1954, pages 1134—1137.

On sait par ailleurs que la [2-(1-pipéridinyl)-(phényl)-méthanone (autre nomenclature: 1-(2-benzoylphényl)-pipéridine] a été décrite et étudiée sur le plan des mécanismes réactionnels de la des hydrogenation, par H. Möhrle et al., Arch. Pharm. 312, pages 219—230 (1979).

On vient de trouver de façon surprenante que de nouveaux dérivés de benzoyl-phényl-pipéridine, qui sont structurellement différents des produits antérieurement connus, sont particulièrement intéressants en thérapeutique en raison de leurs propriétés immunologiques.

Selon l'invention, on préconise un nouveau dérivé de benzoyl-phényl-pipéridine qui est caractérisé en ce qu'il est choisi parmi l'ensemble constitué par

(i) les 2-pipéridinobenzophénones répondant à la formule générale

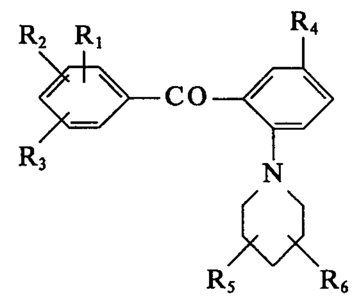

(I)

dans laquelle

$R_1$, $R_2$ et $R_3$, identiques ou différents, représentent chacun l'atome d'hydrogène, un groupe hydroxy, $CF_3$, un halogène, un groupe alkyle en $C_1-C_4$ ou un groupe alkoxy en $C_1-C_4$;
$R_4$ représente l'atome d'hydrogène, un halogène, un groupe $NO_2$, un groupe NR'R" [où R' et R", identiques ou différents, représentent l'atome d'hydrogène, un groupe alkyle en $C_1-C_4$ ou un groupe $CO_2R$ (où R représente un groupe alkyle en $C_1-C_4$ ou un groupe benzyle)];
$R_5$ et $R_6$, identiques ou différents, représentent chacun l'atome d'hydrogène ou un groupe alkyle en $C_1-C_4$, un groupe OH, un groupe phényle ou un groupe benzyle, un au moins des $R_1$, $R_2$, $R_3$, $R_4$, $R_5$ et $R_6$, étant différent de H; et

(ii) leurs sels d'addition d'acide.

Par groupes alkyle inférieur et alkoxy en $C_1-C_4$, on entend ici des restes hydrocarbonés, ramifiés ou linéaires, contenant 1 à 4 atomes de carbone, comme par exemple les groupes méthyle, éthyle, n-propyle, isopropyle, n-butyle, isobutyle, tertiobutyle, et les groupes méthoxy, éthoxy, n-propoxy, isopropoxy, n-butoxy, isobutyloxy et tertiobutyloxy.

Par atome d'halogène, on entend ici un atome de chlore, un atome de brome ou un atome de fluor.

Par sels d'addition d'acide, on entend ici les sels d'addition obtenus par réaction d'une base libre de formule I avec un acide minéral ou organique. Parmi les acides qui conviennent à cet effet, on peut notamment mentionner les acides chlorhydrique, bromhydrique, sulfurique, phosphorique, acétique, fumarique, maléique, oxalique, citrique, tartrique, lactique, malique, benzoïque, succinique, phénylacétique, méthanesulfonique, éthanesulfonique, paratoluènesulfonique.

Parmi les composés de formule I selon l'invention, les produits les plus intéressants sont ceux représentés par les formules I' et I" ci-après, à savoir:

a) les 2-pipéridinobenzophénones de formule

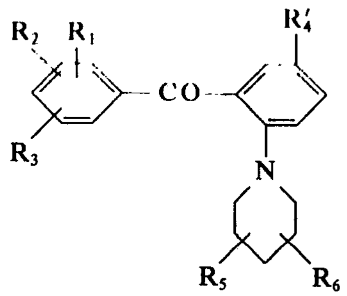

(I')

[dans laquelle $R_1$, $R_2$ et $R_3$, qui peuvent être identiques ou différents, représentent chacun H, F, Cl, Br, OH, $CF_3$, alkyle en $C_1-C_4$ ou alkoxy en $C_1-C_4$; $R'_4$ représente H, F, Cl, Br ou NR'R'' où R' et R'', identiques ou différents, représentent chacun H, alkyle en $C_1-C_4$ ou $CO_2R$ (où R est alkyle en $C_1-C_4$ ou benzyle); $R_5$ et $R_6$, identiques ou différents, représentent chacun H, alkyle en $C_1-C_4$, OH,$C_6H_5$ ou $C_6H_5CH_2$], un au moins des $R_1$, $R_2$, $R_3$, $R_4$, $R_5$ et $R_6$, étant différent de H et leurs sels d'addition d'acide; et

b) les 2-pipéridinobenzophénones de formule

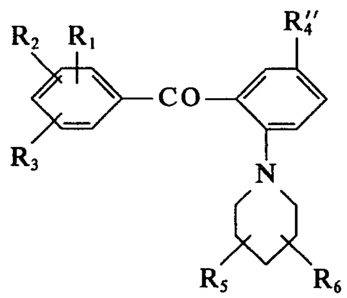

(I'')

[dans laquelle $R_1$, $R_2$ et $R_3$, qui peuvent être identiques ou différents, représentent chacun H, F, Cl, Br, OH, $CF_3$, alkyle en $C_1-C_4$ ou alkoxy en $C_1-C_4$; $R''_4$ représente H, F, Cl, Br, $NO_2$ ou NR'R'' (où R' et R'' sont définis comme indiqué ci-dessus); $R_5$ et $R_6$, identiques ou différents, représentent chacun H, alkyle en $C_1-C_4$, OH, $C_6H_5$ ou $C_6H_5CH_2$; et $R_1$, $R_2$, $R_3$, $R''_4$, $R_5$ et $R_6$ sont tels qu'au moins une des deux conditions A et B suivantes soit satisfaite:

(A) un au moins des $R_1$, $R_2$, $R_3$, $R''_4$, $R_5$ et $R_6$ est différent de H et
(B) $R''_4$ est différent de $NO_2$ quand $R_1=R_2=R_3=R_5=R_6=H$] et leurs sels d'addition d'acide.

Parmi les produits intéressants de formule I qui ont été énoncés ci-dessus, les composés préférés sont ceux où $R_4$ est différent de $NO_2$ et représente avantageusement un groupe $NH_2$. On a en effet observé que, si à faible dose les dérivés nitro de formule I (où $R_4=NO_2$) ont des effets immunostimulants, ils présentent à dose élevée des effets cytotoxiques indésirables. En revanche, on a constaté expérimentalement que les composés de formule I, où $R_4$ est différent de $NO_2$ et représente de façon avantageuse notamment le groupe $NH_2$, sont dépourvus desdits effets cytotoxiques.

Les composés de formule I peuvent être préparés selon une méthode connue en soi par application de mécanismes réactionnels classiques. Le procédé que l'on préconise selon l'invention est caractérisé en ce que

(i) on fait réagir une 2-halogéno-5-nitro-benzophénone de formule

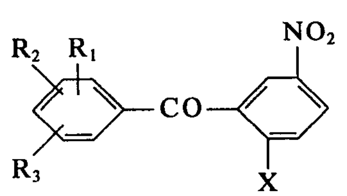

(II)

[dans laquelle $R_1$, $R_2$ et $R_3$ sont définis comme ci-dessus et X représente un atome d'halogène (de préférence Cl ou F pour obtenir des rendements élevés)], avec une pipéridine éventuellement substituée de formule

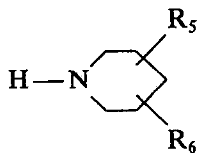

(III)

(dans laquelle $R_5$ et $R_6$ sont définis comme indiqué ci-dessus), pour obtenir un composé nitro de formule

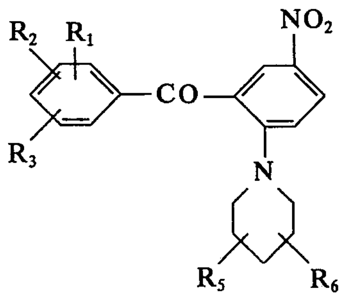

($I_0$)

(ii) si nécessaire, on soumet le composé $I_0$, ainsi obtenu, à une réaction de réduction du groupe nitro en groupe amino pour obtenir un composé amino de formule I où $R_4 = NH_2$, puis, le cas échéant, on soumet ledit dérivé amino à une réaction de désamination, à une alkylation, ou à une réaction de transformation du groupe amino en groupe halogène.

La réaction de la 2-halogéno-5-nitro-benzophénone II avec la pipéridine III est de préférence effectuée dans un solvant organique tel que les hydrocarbures (notamment les hydrocarbures aromatiques), les éthers et les alcools, en présence d'une base minérale ou organique. En pratique, on fera réagir une mole de II avec au moins 1,1 mole de III à une température comprise entre 15°C et la température de reflux du milieu réactionnel.

La réduction du groupe nitro sera conduite dans un solvant organique, de préférence l'éthanol, en présence de fer et d'acide chlorhydrique concentré (HCl 5 N—12 N) et à une température comprise entre 15°C et le point d'ébullition du solvant.

La désamination sera réalisée par diazotation du groupe amino puis substitution du groupe diazonium par l'atome d'hydrogène en présence de cuivre, à une température comprise entre −20°C et +20°C, de préférence à 0°C.

Le groupe diazonium peut également être remplacé par un atome de chlore ou un atome de brome dans les conditions de la réaction de Sandmeyer.

Une amidification du groupe amino sera réalisée par réaction d'un composé de formule (I) où $R_4 = NH_2$ avec un chloroformate d'alkyle ou de benzyle dans un solvant organique, de préférence un hydrocarbure aromatique, comme par exemple le toluène, en présence d'une base minérale, comme par exemple $K_2CO_3$ et à une température comprise entre 0°C et le point d'ébullition du solvant, de préférence à température ambiante (15—20°C).

L'alkylation du groupe amino peut être réalisée par réaction avec un halogénure d'alkyle ou selon la réaction de Eschweiler-Clark dans le cas d'une méthylation.

On a consigné de façon non limitative dans le tableau I ci-après un certain nombre de composés de formule I qui ont été préparés selon les modalités opératoires susvisées.

Selon l'invention, on préconise enfin une composition thérapeutique caractérisée en ce qu'elle renferme, en association avec un excipient physiologiquement acceptable, au moins un composé de formule I ou l'un de ses sels d'addition d'acide non toxiques, en tant que principe actif immunostimulant et immunoadjuvant.

D'autres avantages et caractéristiques de l'invention seront mieux compris à la lecture qui va suivre d'exemples de préparation nullement limitatifs, mais donnés à titre d'illustration.

Preparation I

[2-(4-méthyl-1-pipéridinyl)-5-nitrophényl]-(4-chlorophényl)-méthanone
(Exemple 1; n° de code: 399)

Un mèlange de 0,07 mole (20,7 g) de 2,4'-dichloro-5-nitrobenzophénone, de 0,1 mole (11,4 ml) de 4-méthyl-pipéridine et de 10 g de $K_2CO_3$ dans 100 ml d'éthanol anhydre est chauffé à reflux pendant 3 h. Après refroidissement du milieu réactionnel, le précipité obtenu est filtré, lavé à l'eau, séché puis recristallisé dans l'éthanol. On obtient ainsi 18 g (rendement = 71,7 %) du produit attendu. F = 136°C.

## Preparation II

[2-(4-méthyl-1-pipéridinyl)-5-aminophényl]-(4-chlorophényl)-méthanone
(Exemple 2; n° de code: 442)

Un mélange de 0,02 mole (7,2 g) du produit obtenu selon la préparation I ci-dessus, 0,2 mole (11,2 g) de fer en poudre dans 80 ml du mélange éthanol-eau (90 : 10) v/v et 4 ml d'acide HCl 10 N est porté à reflux pendant 2 h. Après refroidissemet, le milieu réactionnel est filtré, le filtrat est traité par l'acide HCl 10 N, le solvant est évaporé et le produit solide ainsi obtenu est lavé à l'acétate d'éthyle, puis mis en suspension dans l'acétate d'éthyle. Cette suspension est traitée au bicarbonate de sodium et la phase organique est lavée à l'eau puis séchée et évaporée. On obtient 3,9 g de produit solide qui donne, après recristallisation dans l'hexane, 2,9 g (rendement = 44%) du produit attendu. F = 89°C.

### Analyse

Dans le spectre RMN du produit de l'exemple 2 réalisé à 80 MHz dans $CDCl_3$, on observe les déplacements chimiques suivants (exprimés en ppm):

0,70 (intensité = 5); 1,37 (intensité = 3);
2,5 et 2,80 (intensité = 4); 3,58 (intensité = 2);
6,80 (intensité = 3); 7,35 et 7,70 (intensité = 4).

## Preparation III

[2-(4-méthyl-1-pipéridinyl)-5-(N-éthoxycarbonyl-amino)-phényl]-(4-chlorophényl)-méthanone
(Exemple 3; n° de code: 610)

Autre nomenclature

[3-(4-chlorobenzoyl)-4-(4-méthyl-1-pipéridinyl)-phényl]-carbamate d'éthyle

Sous atmosphère d'azote, un mélange de 0,01 mole (3,3 g) du produit obtenu selon la préparation I, de 1,4 g de $K_2CO_3$ et de 0,1 mole (10.8 g) de chloroformate d'éthyle dans le toluène est agité à température ambiante pendant 15 h. Le milieu réactionnel est ensuite étendu à l'eau. La phase aqueuse est extraite à l'acétate d'éthyle puis les phases organiques rassemblées sont lavées à l'eau puis séchées sur sulfate de magnésium. Après évaporation du solvant puis recristallisation dans l'hexane, on obtient 2,9 g (rendement = 72%) du produit attendu. F = 132°C.

## Preparation IV

[2-(4-méthyl-1-pipéridinyl)-phényl]-phényl-méthanone
(Exemple 4; n° de code: 611)

On dissout 0,01 mole (3 g) de [2-(4-méthyl-1-pipéridinyl)-5-aminophényl]-phényl-méthanone (produit de l'exemple 27; n° de code 592) dans de l'éthanol. On refroidit à 0°C puis on ajoute successivement 10 ml d'acide sulfurique et 0,02 mole (1,4 g) de nitrite de sodium. On agite 1 h à 0°C puis on laisse revenir à température ambiante (15—20°C). On ajoute ensuite 0,65 g de cuivre activé et on chauffe le milieu réactionnel à 50°C pendant 1 h. Après hydrolyse et neutralisation, on extrait au chlorure de méthylène puis on lave à l'eau. On sèche puis évapore le solvant. Par recristallisation dans l'éthanol, on obtient 1 g (rendement = 37%) du produit attendu. F = 82°C.

## Preparation V

[2-(4-méthyl-1-pipéridinyl)-5-méthylamino-phényl]-(4-chlorophényl)-méthanone
(Exemple 6; n° de code: 613)

On porte à reflux 5 h un mélange de 0,01 mole (4,8 g) du produit obtenu selon le procédé de la préparation VIII (exemple 26) dans de l'éthanol chlorhydrique (avec HCl 5 N). On évapore l'éthanol, on rince le produit obtenu à l'acétate d'éthyle puis on hydrolyse. Après traitement par la soude, on extrait à l'acétate d'éthyle. On sèche puis évapore le solvant. Le produit obtenu est purefié sur colonne de silice (éluant: hexane-acétone). On obtient 2,5 g (rendement = 70%) du produit attendu. F = 177°C.

Preparation VI

[2-(4-méthyl-1-pipéridinyl)-5-chlorophényl]-(4-chlorophényl)-méthanone
(Exemple 22; n° de code: 633)

On obtient le tétrafluoroborate du diazonium du produit de l'exemple 2 (préparé selon le procédé de la préparation II ci-dessus par réaction à 0°C avec du nitrite de sodium dans de l'acide tétrafluoroborique. Puis 0,02 mole (8,6 g) de ce diazonium est ajoutée goutte à goutte à une solution de 2,75 g de chlorure cuivrique dans le DMSO. 0 n agite 30 min, filtre puis extrait à l'acétate d'éthyle. On sèche et on évapore le solvant. [Concurremment au produit attendu, il se forme le produit de l'exemple 21 (n° de code 634; F 69°C)]. Après purification sur colonne de silice (éluant: benzène) du résidu d'évaporation, on obtient 3 g (rendement = 40 %) du produit attendu. F = 109°C.

Preparation VII

[2-(4-méthyl-1-pipéridinyl)-5-(N-benzyloxycarbonyl-amino)-phényl]-(4-chlorophényl)-méthanone
(exemple 25)

Autre nomenclature
[3-(4-chlorobenzoyl)-4-(4-méthyl-1-pipéridinyl)-phényl]-carbamate de benzyle

En procédant comme indiqué ci-dessus à la préparation III à partir de 0,02 mole (6,6 g) du produit de l'exemple 2 (obtenu selon la préparation II) et de 17 g de chloroformate de benzyle, on obtient 6,6 g (rendement = 71 %) du produit attendu.

Preparation VIII

[2-(4-méthyl-1-pipéridinyl)-5-(N-benzyloxycarbonyl-N-méthyl-amino)-phényl]-
(4-chlorophényl)-méthanone
(exemple 26)

Autre nomenclature
[3-(4-chlorobenzoyl)-4-(4-méthyl-1-pipéridinyl)-phényl]-N-méthyl-carbamate de benzyle

Sous atmosphère d'azote et à 0°C, on agite pendant 30 min dans le THF 0,012 mole (0,3 g) d'hydrure de sodium et 0,012 mole (5,5 g) du produit de l'exemple 25 (obtenu selon le procédé de la préparation VII). On additionne ensuite goutte à goutte 0,18 mole (2,6 g) d'iodure de méthyle dissous dans le THF. On laisse revenir à température ambiante (15—20°C) et continue à agiter pendant 15 h. Après hydrolyse du milieu réactionnel, on extrait à l'acétate d'éthyle puis lave les phases organiques à l'eau. Après séchage et évaporation du solvant, on obtient 5 g (rendement = 85 %) du produit attendu.

Les produits de formule I selon l'invention sont utiles en thérapeutique en tant qu'agents immunostimulants et immunoadjuvants. Ils sont notamment indiqués (i) en immunothérapie cancéreuse, (ii) en tant que stimulants à la résistance antivirale et anti-infectieuse et (iii) dans le traitement des maladies autoimmunes (polyarthrites rhumatoïdes, en particulier).

On a résumé ci-après les essais qui ont été entrepris avec les produits selon l'invention, notamment en ce qui concerne

(A) la stimulation des lymphocytes de souris et
(B) la toxicité.

A — Stimulation des lymphocytes de souris

Des lymphocytes ($5 \times 10^5$ cellules par mesure) extraits de la rate de souris consanguines (DBA/2) sont mis en incubation 36 h à 37°C sous atmosphère d'oxygène à 5 % de $CO_2$ dans un milieu de culture (RPMI 1640) contenant:

- 10 % (en poids) de sérum de veau foetal (100 μl par mesure)
- 10 μl d'une solution de lectines à raison de 5 μg/ml de phytohémaglutinine (PHA) et 40 μg/ml de Pokeweed Mitogen (PWM), et
- la substance à tester.

L'activité est évaluée par mesure de la radio-activité obtenue par incorporation en 24 h de 2 μCi (c'est-à-dire $7,4 \times 10^4$ becquerels) de thymidine tritiée, par rapport à une culture témoin.

Les résultats obtenus sont regroupés dans le tableau II ci-après où on donne la concentration optimale des produits en µg/ml, et où les symboles +, ++ et +++ signifient:

+: stimulation de 0 à 50%,
++: stimulation de 50% à 100%,
+++: stimulation supérieure à 100%.

B — Toxicité

La toxicité (DL-50 et DL-0) a été déterminée par voie i. p. chez la souris selon la technique de S. T. Litchfield décrite dans J. Pharm. Exp. Ther. 96, 99 (1949). Les résultats obtenus ont été consignés dans le tableau III ci-après.

Par ailleurs, le produit de l'exemple 2 (n° de code 442) a été étudié selon le test d'hypersensibilité retardée suivant le protocole opératoire décrit par P. H. Lagrange et al., J. Exp. Med. 139, 1529—1539 (1974). On a observé que, à la dose unique de 5 mg/kg per os administrée chez la souris trois jours avant sensibilisation, le produit de l'exemple 2 conduit à une diminution de moitié de l'hypersensibilité retardée.

La posologie, que l'on préconise en thérapeutique humaine pour les produits selon l'invention, consiste à administrer par voie orale une dose quotidienne de 0,05 mg/kg à 1 mg/kg d'un produit de formule I pendant 7 jours à 3 mois.

Tableau I

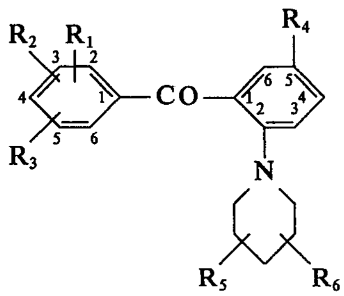

| Exemple | n° de code | $R_1$ | $R_2$ | $R_3$ | $R_4$ | $R_5$ | $R_6$ | F (°C) |
|---|---|---|---|---|---|---|---|---|
| 1 | 399 | 4-Cl | H | H | $NO_2$ | 4-$CH_3$ | H | 136 |
| 2 | 442 | 4-Cl | H | H | $NH_2$ | 4-$CH_3$ | H | 89 |
| 3 | 610 | 4-Cl | H | H | $NHCO_2Et$ | 4-$CH_3$ | H | 132 |
| 4 | 611 | H | H | H | H | 4-$CH_3$ | H | 82 |
| 5 | 612 | 4-Cl | H | H | $N(CH_3)_2$ | 4-$CH_3$ | H | 99,5 |
| 6 | 613 | 4-Cl | H | H | $NHCH_3$ | 4-$CH_3$ | H | 117 |
| 7 | — | 3-Cl | H | H | $NH_2$ | 4-$CH_3$ | H | — |
| 8 | 747 | 2-Cl | H | H | $NH_2$ | 4-$CH_3$ | H | 94 |
| 9 | 697 | 3-$OCH_3$ | 4-$OCH_3$ | H | $NH_2$ | 4-$CH_3$ | H | 132 |
| 10 | — | 3-$OCH_3$ | 4-OH | H | $NH_2$ | 4-$CH_3$ | H | — |
| 11 | — | 3-$OCH_3$ | 4-$OCH_3$ | 5-$OCH_3$ | $NH_2$ | 4-$CH_3$ | H | — |
| 12 | 635 | 4-Cl | H | H | $NH_2$ | H | H | 96 |

Tableau I (suite)

| Exemple | n° de code | $R_1$ | $R_2$ | $R_3$ | $R_4$ | $R_5$ | $R_6$ | F (°C) |
|---|---|---|---|---|---|---|---|---|
| 13 | – | 4-OH | H | H | $NH_2$ | 4-$CH_3$ | H | – |
| 14 | 695 | 4-Cl | H | H | $NH_2$ | 3-$CH_3$ | H | 50 |
| 15 | 655 | 4-$CH_3$ | H | H | $NH_2$ | 4-$CH_3$ | H | 78 |
| 16 | 659 | 2-$CH_3$ | 4-$CH_3$ | H | $NH_2$ | 4-$CH_3$ | H | 90 |
| 17 | 660 | 4-F | H | H | $NH_2$ | 4-$CH_3$ | H | 116 |
| 18 | – | 4-$CF_3$ | H | H | $NH_2$ | 4-$CH_3$ | H | – |
| 19 | 637 | 4-Cl | H | H | $NH_2$ | 3-$CH_3$ | 5-$CH_3$ | 103 |
| 20 | 647 | 4-Cl | H | H | $NH_2$ | 2-$CH_3$ | H | 92 |
| 21 | 634 | 4-Cl | H | H | H | 4-$CH_3$ | H | 69 |
| 22 | 633 | 4-Cl | H | H | Cl | 4-$CH_3$ | H | 109 |
| 23 | 746 | 4-$OCH_3$ | H | H | $NH_2$ | 4-$CH_3$ | H | 64 |
| 24 | 704 | 4-$OCH_3$ | H | H | H | 4-$CH_3$ | H | (a) |
| 25 | – | 4-Cl | H | H | $NHCO_2CH_2C_6H_5$ | 4-$CH_3$ | H | – |
| 26 | – | 4-Cl | H | H | $N(CH_3)CO_2CH_2C_6H_5$ | 4-$CH_3$ | H | – |
| 27 | 592 | H | H | H | $NH_2$ | 4-$CH_3$ | H | 108 |
| 28 | 698 | 3-Cl | 4-Cl | H | $NH_2$ | 4-$CH_3$ | H | 94 |
| 29 | 727 | 3-$CH_3$ | 4-$CH_3$ | 5-$CH_3$ | $NH_2$ | 4-$CH_3$ | H | 127 |
| 30 | 736 | 4-Br | H | H | $NH_2$ | 4-$CH_3$ | H | 105 |
| 31 | 757 | 4-Cl | H | H | $NH_2$ | 4-$C(CH_3)_3$ | H | 109 |
| 32 | – | 4-Cl | H | H | $NH_2$ | 4-$C_2H_5$ | H | 92 |
| 33 | – | 4-Cl | H | H | $NH_2$ | 4-n-$C_4H_9$ | H | 103 |
| 34 | – | 4-Cl | H | H | $NH_2$ | 4-$CH_2C_6H_5$ | H | 109 |
| 35 | – | 4-Cl | H | H | $NH_2$ | 4-$C_6H_5$ | H | 144 |
| 36 | – | 4-Cl | H | H | $NH_2$ | 4-OH | H | 71 |
| 37 | – | 2-Cl | 4-Cl | H | $NH_2$ | 4-$CH_3$ | H | 117 |

Note:

(a): huile, $n_D^{20} = 1,5950$

Tableau II

Stimulation in vitro des lymphocytes des souris.

| Exemple | n° de code | Concentration optimale (μg/ml) | Stimulation |
|---|---|---|---|
| 1 | 399 | 0,2 | ++ |
| 2 | 442 | 1 | +++ |
| 3 | 610 | 0,2 | ++ |
| 5 | 612 | 0,2 | + |
| 6 | 613 | 0,2 | + |
| 8 | 747 | 0,2 | + |
| 9 | 697 | 0,4 | + |
| 12 | 635 | 0,2 | + |
| 14 | 695 | 1 | ++ |
| 15 | 655 | 5 | ++ |
| 16 | 659 | 1 | ++ |
| 17 | 660 | 1 | + |
| 19 | 637 | 1 | ++ |
| 20 | 647 | 1 | +++ |
| 22 | 633 | 1 | + |
| 23 | 746 | 1 | ++ |
| 24 | 704 | 0,2 | + |
| 27 | 592 | 5 | + |
| 28 | 698 | 1,25 | ++ |
| 29 | 727 | 2,5 | + |

Tableau III

Toxicité

| Exemple | n° de code | Toxicité i. p. souris (mg/kg) | |
|---|---|---|---|
| 1 | 399 | DL-0 | > 800 |
| 2 | 442 | DL-50 | = 1300 |
| 3 | 610 | DL-0 | > 800 |
| 4 | 611 | DL-0 | > 800 |
| 5 | 612 | DL-0 | > 800 |
| 6 | 613 | DL-0 | > 800 |
| 8 | 747 | DL-0 | > 800 |
| 9 | 697 | DL-0 | > 800 |
| 12 | 635 | DL-0 | > 800 |
| 15 | 655 | DL-0 | > 800 |
| 16 | 659 | DL-50 | = 750 |
| 17 | 660 | DL-0 | > 800 |
| 19 | 637 | DL-0 | > 800 |
| 20 | 647 | DL-0 | > 800 |
| 21 | 634 | DL-50 | = 1800 |
| 22 | 633 | DL-50 | = 1800 |
| 23 | 746 | DL-0 | > 800 |
| 24 | 704 | DL-0 | > 800 |
| 27 | 592 | DL-0 | > 800 |
| 28 | 698 | DL-0 | > 800 |
| 29 | 727 | DL-0 | > 800 |
| 30 | 736 | DL-0 | > 800 |
| 31 | 757 | DL-0 | > 800 |

**Revendications**

**Pour les états contractants: BE, CH, DE, FR, GB, IT, LI, LU, NL, SE.**

1. Dérivé de benzoyl-phényl-pipéridine, caractérisé en ce qu'il est choisi parmi l'ensemble constitué par:

(i) les 2-pipéridinobenzophénones répondant à la formule générale:

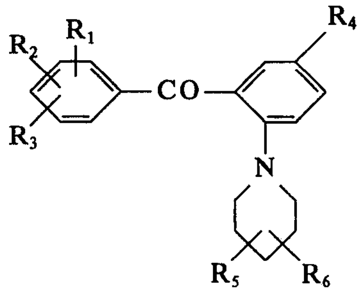

(I)

dans laquelle:

- $R_1$, $R_2$ et $R_3$, identiques ou différents, représentent chacun l'atome d'hydrogène, un groupe hydroxy, $CF_3$, un halogène, un groupe alkyle en $C_1-C_4$ ou un groupe alkoxy en $C_1-C_4$;
- $R_4$ représente l'atome d'hydrogène, un halogène, un groupe $NO_2$, un groupe NR'R" [où R' et R", identiques ou différents, représentent l'atome d'hydrogène, un groupe alkyle en $C_1-C_4$ ou un groupe $CO_2R$ (où R représente un groupe alkyle en $C_1-C_4$ ou un groupe benzyle)];
- $R_5$ et $R_6$, identiques ou différents, représentent chacun l'atome d'hydrogène, un groupe alkyle en $C_1-C_4$, un groupe OH, un groupe phényle ou un groupe benzyle, un au moins des $R_1$, $R_2$, $R_3$, $R_4$, $R_5$ et $R_6$ étant différent de H; et

(ii) leurs sels d'addition d'acide.

2. Dérivé de benzoyl-phényl-pipéridine, caractérisé en ce qu'il est choisi parmi l'ensemble constitué par:

(i) les 2-pipéridinobenzophénones répondant à la formule générale:

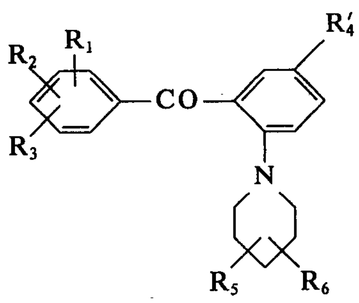

(I')

dans laquelle:

- $R_1$, $R_2$ et $R_3$, identiques ou différents, représentent chacun H, F, Cl, Br, OH, $CF_3$, un groupe alkyle en $C_1-C_4$ ou alkoxy en $C_1-C_4$;
- $R'_4$ représente H, Cl, Br, F ou NR'R" [où R' et R", identiques ou différents, représentent chacun H, un groupe alkyle en $C_1-C_4$ ou un groupe $CO_2R$ (où R est un groupe alkyle en $C_1-C_4$ ou un groupe benzyle)];
- $R_5$ et $R_6$, identiques ou différents, représentent chacun H, un groupe alkyle en $C_1-C_4$, un groupe OH, un groupe phényle ou un groupe benzyle, un au moins des $R_1$, $R_2$, $R_3$, $R_4$, $R_5$ et $R_6$ étant différent de H; et

(ii) leurs sels d'addition d'acide.

3. Dérivé de benzoyl-phényl-pipéridine, caractérisé en ce qu'il est choisi parmi l'ensemble constitué par:

(i) les 2-pipéridinobenzophénones répondant à la formule générale:

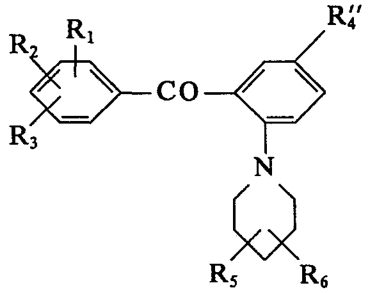

(I'')

dans laquelle:

- $R_1$, $R_2$ et $R_3$, identiques ou différents, représentent chacun H, F, Cl, Br, OH, $CF_3$, un groupe alkyle en $C_1-C_4$ ou alkoxy en $C_1-C_4$ ;
- $R_4''$ représente H, Cl, Br, F, $NO_2$ ou NR'R'' [où R' et R'', identiques ou différents, représentent chacun H, un groupe alkyle en $C_1-C_4$ ou un groupe $CO_2R$ (où R est un groupe alkyle en $C_1-C_4$ ou benzyle)];
- $R_5$ et $R_6$, identiques ou différents, représentent chacun H, un groupe alkyle en $C_1-C_4$, un groupe OH, un groupe phényle ou un groupe benzyle, les groupes $R_1$, $R_2$, $R_3$, $R_4''$, $R_5$ et $R_6$ étant tels qu'au moins une des deux conditions (A) et (B) suivantes soit satisfaite: (A) au moins un des $R_1$, $R_2$, $R_3$, $R_4''$, $R_5$ et $R_6$ est différent de H, et (B) $R_4''$ est différent de $NO_2$ quand $R_1 = R_2 = R_3 = R_5 = R_6 = H$; et

(ii) leurs sels d'addition d'acide.

4. [2-(4-Méthyl-1-pipéridinyl)-5-nitrophényl]-(4-chlorophényl)-méthanone et ses sels d'addition d'acide.

5. [2-(4-Méthyl-1-pipéridinyl)-5-aminophényl]-(4-chlorophényl)-méthanone et ses sels d'addition d'acide.

6. [2-(3-Méthyl-1-pipéridinyl)-5-aminophényl]-(4-chlorophényl)-méthanone et ses sels d'addition d'acide.

7. [2-(2-Méthyl-1-pipéridinyl)-5-aminophényl]-(4-chlorophényl)-méthanone et ses sels d'addition d'acide.

8. Composition thérapeutique, caractérisée en ce qu'elle renferme, en association avec un excipient physiologiquement acceptable, au moins un dérivé de formule (I) selon la revendication 1 ou l'un de ses sels d'addition d'acide non toxique.

9. Composition thérapeutique, caractérisée en ce qu'elle renferme, en association avec un excipient physiologiquement acceptable, au moins un dérivé de formule (I') selon la revendication 2 ou l'un de ses sels d'addition d'acide non toxique.

10. Procédé de préparation d'un composé de formule (I) selon la revendication 1, caractérisé en ce que:

(i) on fait réagir une 2-halogéno-5-nitro-benzophénone de formule:

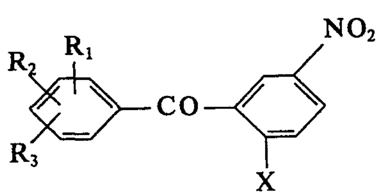

(II)

(dans laquelle $R_1$, $R_2$ et $R_3$ sont définis comme ci-dessus et X représente un atome d'halogène) avec une pipéridine éventuellement substituée de formule:

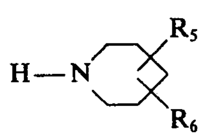

(III)

(dans laquelle $R_5$ et $R_6$ sont définis comme ci-dessus), pour obtenir un composé nitro de formule:

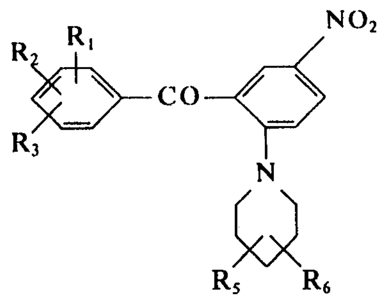

($I_0$)

(ii) si nécessaire, on soumet le composé $I_0$, ainsi obtenu, à une réaction de réduction du groupe nitro en groupe amino pour obtenir un composé amino de formule (I) où $R_4 = NH_2$, puis, le cas échéant, on soumet ledit dérivé amino à une réaction de désamination, à une alkylation ou à une réaction de transformation du groupe amino en groupe halogène.

**Revendications pour l'état contractant: AT.**

1. Procédé de préparation d'un dérivé de benzoyl-phényl-pipéridine choisi parmi l'ensemble des 2-pipéridinobenzophénones répondant à la formule générale

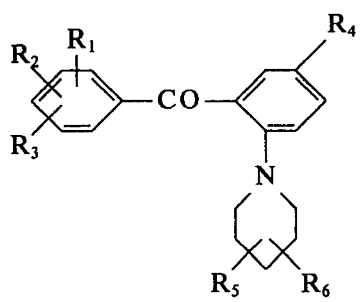

(I)

dans laquelle:

- $R_1$, $R_2$ et $R_3$, identiques ou différents, représentent chacun l'atome d'hydrogène, un groupe hydroxy, $CF_3$, un halogène, un groupe alkyle en $C_1-C_4$ ou un groupe alkoxy en $C_1-C_4$;
- $R_4$ représente l'atome d'hydrogène, un halogène, un groupe $NO_2$, un groupe NR'R" [où R' et R", identiques ou différents, représentent l'atome d'hydrogène, un groupe alkyle en $C_1-C_4$ ou un groupe $CO_2R$ (où R représente un groupe alkyle en $C_1-C_4$ ou un groupe benzyle)];
- $R_5$ et $R_6$, identiques ou différents, représentent chacun l'atome d'hydrogène, un groupe alkyle en $C_1-C_4$, un groupe OH, un groupe phényle ou un groupe benzyle, un au moins des $R_1$, $R_2$, $R_3$, $R_4$, $R_5$ et $R_6$ étant différent de H;

et leurs sels d'addition d'acide, ledit procédé étant caractérisé en ce que

(i) on fait réagir une 2-halogéno-5-nitro-benzophénone de formule:

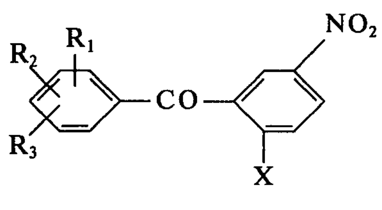

(II)

(dans laquelle $R_1$, $R_2$ et $R_3$ sont définis comme ci-dessus et X représente un atome d'halogène) avec une pipéridine éventuellement substituée de formule:

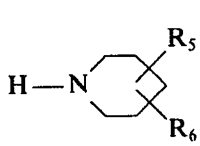

(III)

(dans laquelle $R_5$ et $R_6$ sont définis comme ci-dessus), pour obtenir un composé nitro de formule:

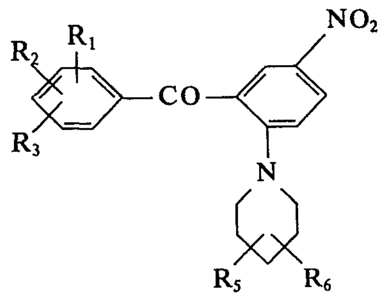

($I_0$)

(ii) si nécessaire, on soumet le composé $I_0$ ainsi obtenu à une réaction de réduction du groupe nitro en groupe amino pour obtenir un composé amino de formule (I) où $R_4 = NH_2$, puis, le cas échéant, on soumet ledit dérivé amino à une réaction de désamination, à une alkylation ou à une réaction de transformation du groupe amino en groupe halogène.

2. Procédé selon la revendication 1, caractérisé en ce que $R_1$ = 4-Cl; $R_4 = NO_2$; $R_5 = 4\text{-}CH_3$; et $R_2 = R_3 = R_6 = H$.

3. Procédé selon la revendication 1, caractérisé en ce que $R_1$ = 4-Cl; $R_4 = NH_2$; $R_5 = 4\text{-}CH_3$; et $R_2 = R_3 = R_6 = H$.

4. Procédé selon la revendication 1, caractérisé en ce que $R_1$ = 4-Cl; $R_4 = NH_2$; $R_5 = 3\text{-}CH_3$; et $R_2 = R_3 = R_6 = H$.

5. Procédé selon la revendication 1, caractérisé en ce que $R_1$ = 4-Cl; $R_4 = NH_2$; $R_5 = 2\text{-}CH_3$; et $R_2 = R_3 = R_6 = H$.

**Patentansprüche für die Vertragsstaaten: BE, CH, DE, FR, GB, IT, LI, LU, NL, SE.**

1. Benzoylphenyl-piperidin-Derivat, dadurch gekennzeichnet, daß es ausgewählt ist aus der Gruppe, bestehend aus:

(i) den 2-Piperidinobenzophenonen der allgemeinen Formel

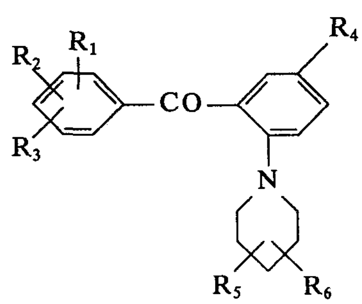

(I)

worin
$R_1$, $R_2$ und $R_3$ gleich oder verschieden sind und jeweils Wasserstoff, Hydroxy, $CF_3$, Halogen, eine $C_1$—$C_4$-Alkylgruppe oder eine $C_1$—$C_4$-Alkoxygruppe bedeuten;
$R_4$ für Wasserstoff, Halogen, Nitro, eine Gruppe NR'R" [in der R' und R" gleich oder verschieden sind und Wasserstoff, eine $C_1$—$C_4$-Alkylgruppe oder eine Gruppe $CO_2R$ darstellen (wobei R eine $C_1$—$C_4$-Alkylgruppe oder eine Benzylgruppe bedeutet)] steht;
$R_5$ und $R_6$ gleich oder verschieden sind und jeweils Wasserstoff, eine $C_1$—$C_4$-Alkylgruppe, eine Hydroxygruppe, eine Phenylgruppe oder eine Benzylgruppe repräsentieren, wobei wenigstens einer der Reste $R_1$, $R_2$, $R_3$, $R_4$, $R_5$ und $R_6$ von Wasserstoff verschieden ist; und

(ii) deren Säureadditionssalzen.

2. Benzoylphenyl-piperidin-Derivat, dadurch gekennzeichnet, daß es ausgewählt ist aus der Gruppe, bestehend aus:

(i) den 2-Piperidinobenzophenonen der allgemeinen Formel

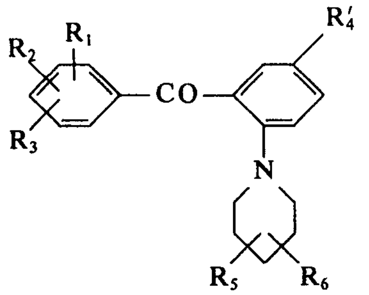

(I')

worin $R_1$, $R_2$ und $R_3$ gleich oder verschieden sind und jeweils H, F, Cl, Br, OH, $CF_3$, eine $C_1$—$C_4$-Alkylgruppe oder $C_1$—$C_4$-Alkoxy bedeuten;
$R'_4$ für H, Cl, Br, F oder NR'R" [worin R' und R" gleich oder verschieden sind und jeweils H, eine $C_1$—$C_4$-Alkylgruppe oder eine Gruppe $CO_2R$ darstellen (wobei R eine $C_1$—$C_4$-Alkylgruppe oder eine Benzylgruppe ist)] steht;
$R_5$ und $R_6$ gleich oder verschieden sind und jeweils H, eine $C_1$—$C_4$-Alkylgruppe, eine Gruppe OH, eine Phenylgruppe oder eine Benzylgruppe repräsentieren, wobei wenigstens einer der Reste $R_1$, $R_2$, $R_3$, $R_4$, $R_5$ und $R_6$ von H verschieden ist; und
(ii) deren Säureadditionssalzen.

3. Benzoylphenyl-piperidin-Derivat dadurch gekennzeichnet, daß es ausgewählt ist aus der Gruppe, bestehend aus

(i) den 2-Piperidinobenzophenonen der allgemeinen Formel

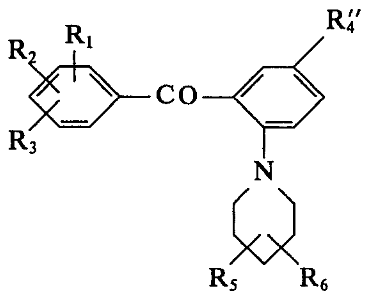

(I")

worin $R_1$, $R_2$ und $R_3$ gleich oder verschieden sind und jeweils H, F, Cl, Br, OH, $CF_3$, eine $C_1$—$C_4$-Alkylgruppe oder $C_1$—$C_4$-Alkoxy bedeuten;
$R''_4$ H, Cl, Br, F, $NO_2$ oder NR'R" darstellt [worin R' und R" gleich oder verschieden sind und jeweils H, eine $C_1$—$C_4$-Alkylgruppe oder eine Gruppe $CO_2R$ (wobei R eine $C_1$—$C_4$-Alkylgruppe oder Benzyl ist) repräsentieren];
$R_5$ und $R_6$ gleich oder verschieden sind und jeweils für H, eine $C_1$—$C_4$-Alkylgruppe, eine Gruppe OH, eine Phenylgruppe oder eine Benzylgruppe stehen, wobei die Gruppen $R_1$, $R_2$, $R_3$, $R''_4$, $R_5$ und $R_6$ so gewählt sind, daß wenigstens eine der beiden folgenden Bedingungen (A) und (B) erfüllt ist:
(A) wenigstens eines von $R_1$, $R_2$, $R_3$, $R''_4$, $R_5$ und $R_6$ ist von H verschieden und
(B) $R''_4$ ist von $NO_2$ verschieden, wenn $R_1 = R_2 = R_3 = R_5 = R_6 = H$; und
(ii) deren Säureadditionssalzen.

4. [2-(4-Methyl-1-piperidinyl)-5-nitrophenyl]-(4-chlorphenyl)-methanon und dessen Säureadditionssalze.
5. [2-(4-Methyl-1-piperidinyl)-5-aminophenyl]-(4-chlorphenyl)-methanon und dessen Säureadditionssalze.
6. [2-(3-Methyl-1-piperidinyl)-5-aminophenyl]-(4-chlorphenyl)-methanon und dessen Säureadditionssalze.
7. [2-(2-Methyl-1-piperidinyl)-5-aminophenyl]-(4-chlorphenyl)-methanon und dessen Säureadditionssalze.
8. Therapeutische Zusammensetzung, dadurch gekennzeichnet, daß sie gemeinsam mit einem physiologisch akzeptablen Exzipienten wenigstens ein Derivat der Formel (I) nach Anspruch 1 oder eines von dessen Additionssalzen mit einer nicht toxischen Säure umfaßt.
9. Therapeutische Zusammensetzung, dadurch gekennzeichnet, daß sie gemeinsam mit einem physiologisch akzeptablen Exzipienten wenigstens ein Derivat der Formel (I') nach Anspruch 2 oder eines von dessen Additionssalzen mit einer nicht toxischen Säure umfaßt.

10. Verfahren zur Herstellung einer Verbindung der Formel (I) nach Anspruch 1, dadurch gekennzeichnet, daß man:

(i) ein 2-Halogen-5-nitro-benzophenon der Formel

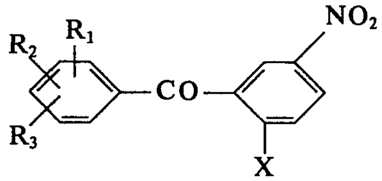

(II)

worin $R_1$, $R_2$ und $R_3$ wie oben definiert sind und X für Halogen steht, mit einem gegebenenfalls substituierten Piperidin der Formel

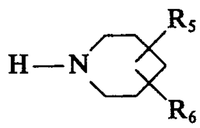

(III)

worin $R_5$ und $R_6$ obige Bedeutung haben, zu einer Nitroverbindung der Formel

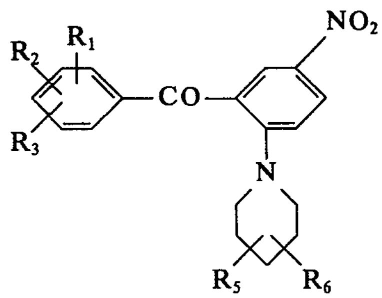

($I_0$)

umsetzt,

(ii) notwendigenfalls die so erhaltene Verbindung $I_0$ einer Reduktionsreaktion der Nitrogruppe zu einer Aminogruppe unterwirft, um eine Aminoverbindung der Formel (I) zu erhalten, worin $R_4 = NH_2$, worauf man gewünschtenfalls diese Aminoverbindung einer Desaminierungsreaktion, einer Alkylierung oder einer Umwandlungsreaktion der Aminogruppe zu einer Halogengruppe unterzieht.

**Patentansprüche für den Vertragsstaat: AT**

1. Verfahren zur Herstellung eines Benzoylphenyl-piperidin-Derivates, ausgewählt aus der Gruppe von 2-Piperidinobenzophenonen der allgemeinen Formel

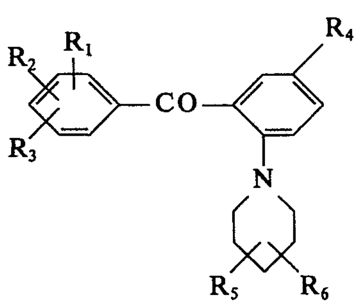

(I)

worin

$R_1$, $R_2$ und $R_3$ gleich oder verschieden sind und jeweils Wasserstoff, Hydroxy, $CF_3$, Halogen, eine $C_1$—$C_4$-Alkylgruppe oder eine $C_1$—$C_4$-Alkoxygruppe bedeuten;

$R_4$ für Wasserstoff, Halogen, Nitro, eine Gruppe NR'R'' [in der R' und R'' gleich oder verschieden sind und Wasserstoff, eine $C_1$—$C_4$-Alkylgruppe oder eine Gruppe $CO_2R$ darstellen (wobei R eine $C_1$—$C_4$-Alkylgruppe oder eine Benzylgruppe bedeutet)] steht;

$R_5$ und $R_6$ gleich oder verschieden sind und jeweils Wasserstoff, eine $C_1$—$C_4$-Alkylgruppe, eine Hydroxygruppe, eine Phenylgruppe oder eine Benzylgruppe repräsentieren, wobei wenigstens einer der Reste $R_1$, $R_2$, $R_3$, $R_4$, $R_5$ und $R_6$ von Wasserstoff verschieden ist;

sowie deren Säureadditionssalzen, welches Verfahren dadurch gekennzeichnet ist, daß man

(i) ein 2-Halogen-5-nitro-benzophenon der Formel

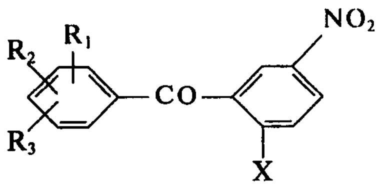

(II)

worin $R_1$, $R_2$ und $R_3$ wie oben definiert sind und X für Halogen steht, mit einem gegebenenfalls substituierten Piperidin der Formel

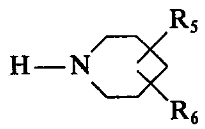

(III)

worin $R_5$ und $R_6$ obige Bedeutung haben, zu einer Nitroverbindung der Formel

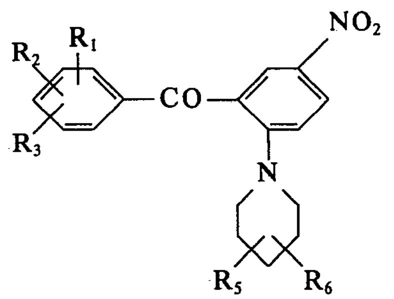

($I_0$)

umsetzt,

(ii) notwendigenfalls die so erhaltene Verbindung $I_0$ einer Reduktionsreaktion der Nitrogruppe zu einer Aminogruppe unterwirft, um eine Aminoverbindung der Formel (I) zu erhalten, worin $R_4 = NH_2$, worauf man gewünschtenfalls diese Aminoverbindung einer Desaminierungsreaktion, einer Alkylierung oder einer Umwandlungsreaktion der Aminogruppe zu einer Halogengruppe unterzieht.

2. Verfahren nach Anspruch 1, dadurch gekennzeichnet, daß $R_1$ = 4-Cl; $R_4 = NO_2$; $R_5$ = 4-$CH_3$; und $R_2 = R_3 = R_6$ = H.

3. Verfahren nach Anspruch 1, dadurch gekennzeichnet, daß $R_1$ = 4-Cl; $R_4 = NH_2$; $R_5$ = 4-$CH_3$; und $R_2 = R_3 = R_6$ = H.

4. Verfahren nach Anspruch 1, dadurch gekennzeichnet, daß $R_1$ = 4-Cl; $R_4 = NH_2$; $R_5$ = 3-$CH_3$; und $R_2 = R_3 = R_6$ = H.

5. Verfahren nach Anspruch 1, dadurch gekennzeichnet, daß $R_1$ = 4-Cl; $R_4 = NH_2$; $R_5$ = 2-$CH_3$; und $R_2 = R_3 = R_6$ = H.

**Claims for the Contracting States: BE, CH, DE, FR, GB, IT, LI, LU, NL, SE**

1. Benzoyl-phenyl-piperidine derivative, characterized in that it is selected from the group constituted by:

(i) the 2-piperidinobenzophenones of general formula:

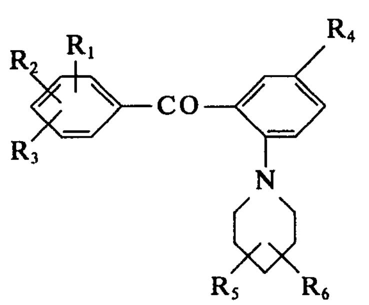

(I)

wherein:

- $R_1$, $R_2$ and $R_3$, either identical or different, each represent the hydrogen atom, a hydroxy group, $CF_3$, a halogen, a $C_1$—$C_4$ alkyl group or a $C_1$—$C_4$ alkoxy group;
- $R_4$ represents the hydrogen atom, a halogen, a $NO_2$ group, a NR'R" group [wherein R' and R", either identical or different, each represent the hydrogen atom, a $C_1$—$C_4$ alkyl group, or a $CO_2R$ group (wherein R represents a $C_1$—$C_4$ alkyl group, or a benzyl group)];
- $R_5$ and $R_6$, either identical or different, each represent the hydrogen atom, a $C_1$—$C_4$ alkyl group, a OH group, a phenyl group or a benzyl group, one at least of $R_1$, $R_2$, $R_3$, $R_4$, $R_5$ and $R_6$ being different from H; and

(ii) their acid addition salts.

2. Benzoyl-phenyl-piperidine derivative, characterized in that it is selected from the group constituted by:

(i) the 2-piperidinobenzophenone of general formula:

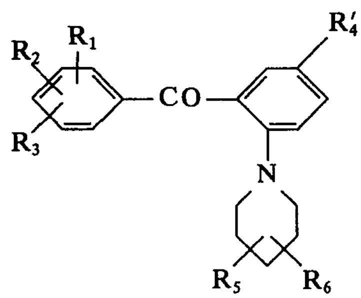

(I')

wherein:

- $R_1$, $R_2$ and $R_3$, either identical or different, each represent H, F, Cl, Br, OH, $CF_3$, a $C_1$—$C_4$ alkyl or $C_1$—$C_4$ alkoxy group;
- $R'_4$ represents H, Cl, Br, F or NR'R" [wherein R' and R", either identical or different, each represent H, a $C_1$—$C_4$ alkyl group or a $CO_2R$ group (wherein R is a $C_1$—$C_4$ alkyl group or a benzyl group)];
- $R_5$ and $R_6$, either identical or different, each represent H, a $C_1$—$C_4$ alkyl group, a OH group, a phenyl group or a benzyl group, one at least of $R_1$, $R_2$, $R_3$, $R_4$, $R_5$ and $R_6$ being different from H; and

(ii) their acid addition salts.

3. Benzoyl-phenyl-piperidine derivative, characterized in that it is selected from the group constituted by:

(i) the 2-piperidinobenzophenones of the general formula:

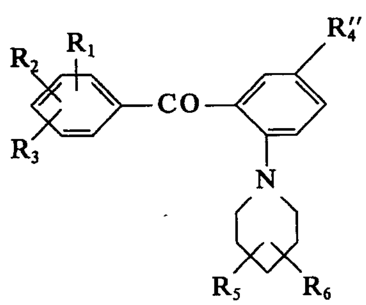

(I")

wherein:

- $R_1$, $R_2$ and $R_3$, either identical or different, each represent H, F, Cl, Br, OH, $CF_3$, a $C_1$—$C_4$ alkyl or $C_1$—$C_4$ alkoxy group;
- $R''_4$ represents H, Cl, Br, F, $NO_2$ or NR'R" [wherein R' and R", either identical or different, each represent H, a $C_1$—$C_4$ alkyl group or a $CO_2R$ group (wherein R is a $C_1$—$C_4$ alkyl or benzyl group)];
- $R_5$ and $R_6$, either identical or different, each represent H, a $C_1$—$C_4$ alkyl group, a OH group, a

phenyl or benzyl group, the groups $R_1$, $R_2$, $R_3$, $R''_4$, $R_5$ and $R_6$ being such that at least one of the two following conditions (A) and (B) is met: (A) at least one of the $R_1$, $R_2$, $R_3$, $R''_4$, $R_5$ and $R_6$ is different from H, and (B) $R''_4$ is different from $NO_2$ when $R_1 = R_2 = R_3 = R_5 = R_6 = H$; and

(ii) their acid additions salts.

4. [2-(4-Methyl-1-piperidinyl)-5-nitrophenyl]-(4-chlorophenyl)-methanone and its acid addition salts.
5. [2-(4-Methyl-1-piperidinyl)-5-aminophenyl]-(4-chlorophenyl)-methanone and its acid addition salts.
6. [2-(3-Methyl-1-piperidinyl)-5-aminophenyl]-(4-chlorophenyl)-methanone and its acid addition salts.
7. [2-(2-Methyl-1-piperidinyl)-5-aminophenyl]-(4-chlorophenyl)-methanone and its acid addition salts.
8. Therapeutical composition, characterized in that it contains, in association with a physiologically acceptable excipient, at least one derivative of formula (I) according to claim 1 or one of its nontoxic addition salts.
9. Therapeutical composition, characterized in that it contains, in association with a physiologically acceptable excipient, at least one derivative of formula (I') according to claim 2 or one of its nontoxic acid addition salts.
10. Process for the preparation of a compound of formula (I) according to claim 1, characterized in that:

(i) a 2-halogeno-5-nitro-benzophenone of formula:

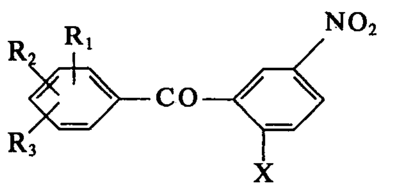

(II)

(wherein $R_1$, $R_2$ and $R_3$ are defined as above and X represents a halogen atom) is reacted with an optionally substituted piperidine of formula:

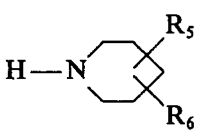

(III)

(wherein $R_5$ and $R_6$ are defined as above), to obtain a nitro compound of formula:

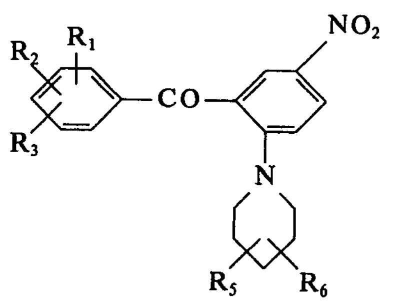

($I_0$)

(ii) if necessary, the compound $I_0$, thus obtained, is subjected to a reduction reaction of the nitro group into an amino to obtain an amino-compound of formula (I) wherein $R_4 = NH_2$, then, optionally, said amino derivative is subjected to a desaminating reaction, to an alkylation or a conversion reaction of the amino-group into a halogen group.

**Claims for the Contracting State: AT**

1. Process for the preparation of a benzoyl-phenyl-piperidine derivative selected from the group of 2-piperidinobenzophenones of the general formula:

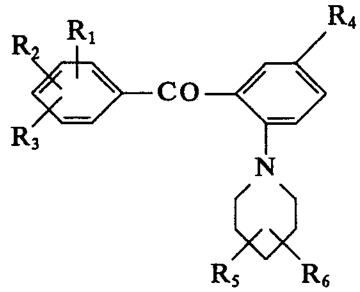

(I)

wherein:

- $R_1$, $R_2$ and $R_3$, either identical or different, each represent the hydrogen atom, a hydroxy group, $CF_3$, a halogen, a $C_1$—$C_4$ alkyl group or a $C_1$—$C_4$ alkoxy group;
- $R_4$ represents the hydrogen atom, a halogen, a $NO_2$ group, a NR'R'' group [wherein R' and R'', either identical or different, represent the hydrogen atom, a $C_1$—$C_4$ alkyl group or a $CO_2R$ group (wherein R represents a $C_1$—$C_4$ alkyl group or a benzyl group)];
- $R_5$ and $R_6$, either identical or different, each represent the hydrogen atom, a $C_1$—$C_4$ alkyl group, a OH group, a phenyl group or a benzyl group, and at least one of $R_1$, $R_2$, $R_3$, $R_4$, $R_5$ and $R_6$ being different from H;

and their acid addition salts, said process being characterized in that

(i) a 2-halogeno-5-nitro-benzophenone of formula:

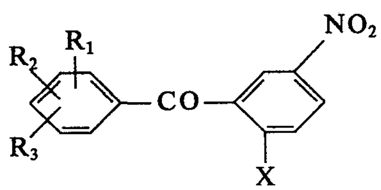

(II)

(wherein $R_1$, $R_2$ and $R_3$ are defined as above and X represents a halogen atom) is reacted with an optionally substituted piperidine of formula:

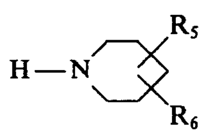

(III)

(wherein $R_5$ and $R_6$ are defined as above), to obtain a nitro compound of formula:

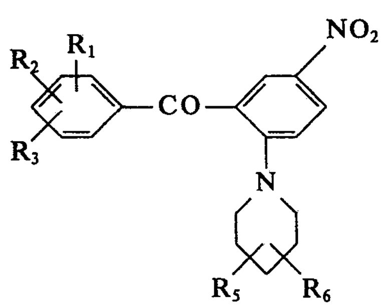

($I_0$)

(ii) if necessary, the compound $I_0$, thus obtained, is subjected to a reduction reaction of the nitro group into an amino group to obtain an amino compound of formula (I) wherein $R_4 = NH_2$, then, optionally, said amino derivative is subjected to a desaminating reaction, to an alkylation or to a conversion reaction of the amino group into a halogen group.

2. Process according to claim 1, characterized in that $R_1$ = 4-Cl; $R_4$ = $NO_2$; $R_5$ = 4-$CH_3$; and $R_2 = R_3 = R_6$ = H.

3. Process according to claim 1, characterized in that $R_1$ = 4-Cl; $R_4$ = $NH_2$; $R_5$ = 4-$CH_3$; and $R_2 = R_3 = R_6$ = H.

4. Process according to claim 1, characterized in that $R_1$ = 4-Cl; $R_4$ = $NH_2$; $R_5$ = 3-$CH_3$; and $R_2 = R_3 = R_6$ = H.

5. Process according to claim 1, characterized in that $R_1$ = 4-Cl; $R_4$ = $NH_2$; $R_5$ = 2-$CH_3$; and $R_2 = R_3 = R_6$ = H.